# EUROPEAN PATENT APPLICATION

(11) **EP 3 406 198 A1**
(43) Date of publication of application: **28.11.2018**
(21) Application number: 17741552.8
(22) Date of filing: 20.01.2017
(51) Int. Cl.: A61B 8/06

(54) **ULTRASONIC OBSERVATION DEVICE, METHOD FOR OPERATING ULTRASONIC OBSERVATION DEVICE, AND PROGRAM FOR OPERATING ULTRASONIC OBSERVATION DEVICE**

(30) Priority: 22.01.2016 JP 2016010711
(71) Applicant: Olympus Corporation, Hachioji-shi Tokyo 192-8507 (JP)
(72) Inventor: MISONO, Kazuhiro, Hachioji-shi Tokyo 192-8507 (JP)
(74) Representative: Gunzelmann, Rainer
(86) International application number: PCT/JP2017/001971
(87) International publication number: WO 2017/126675

(57) **Abstract**

An ultrasound observation device according to the present invention is an ultrasound observation device configured to generate Doppler information based on multiple sets of scan data acquired during sequential alternating scan in which ultrasound waves are sequentially transmitted in multiple depth directions and in which the ultrasound waves are repeatedly transmitted in this sequential order, and it includes: a calculation unit configured to perform comparison operation on first scan data acquired by transmitting the ultrasound waves in a depth direction for first scan during the sequential alternating scan and second scan data acquired by transmitting the ultrasound waves for a second time during the sequential alternating scan, the second scan data being acquired by transmitting the ultrasound waves in a depth direction identical to the depth direction for the first scan; and a sequential-alternating scan controller configured to control a repetition frequency based on a calculation result of the calculation unit.

## Description

### Field

The present invention relates to an ultrasound observation device that observes a tissue that is an observation target by using ultrasound waves, a method for operating the ultrasound observation device, and a program for operating the ultrasound observation device.

### Background

Ultrasound waves are sometimes used to observe the characteristics of a living tissue or material that is an observation target. Specifically, ultrasound waves are transmitted to the observation target and predetermined signal processing is performed on ultrasound echoes reflected by the observation target so that information about the characteristics of the observation target is acquired.

Ultrasound endoscopes with an ultrasound transducer provided in the distal end of an insertion unit are used for diagnosis of living tissues, or the like, inside a body to which ultrasonic waves are applied. An operator such as a doctor inserts an insertion unit into the inside of a body and then operates an operating unit at hand so that ultrasound echoes are acquired by the ultrasound transducer and diagnosis is conducted based on information (ultrasound images) based on ultrasound echoes. In ultrasound diagnosis systems, ultrasound images are displayed in various operation modes such as flow mode, elasto mode, or contrast mode, for more detailed diagnosis, a higher degree of certainty of results in a comprehensive way from a different point of view of diagnosis, or the like. Specifically, the region of interest is set as the basis on a B-mode image, an operation mode image is generated which two-dimensionally represents additional information obtained by performing calculation, or the like, corresponding to the set operation mode on the region of interest, and it is superimposed on the B-mode image and is displayed on the monitor.

Among the above-described modes, the flow mode is a mode to detect blood by analyzing Doppler shift and superimpose two-dimensional information in which the presence or absence of a blood flow or the direction of a blood flow is color-coded. In the flow mode, scanning is conducted multiple times in the identical depth direction and, in accordance with the amount of change in the amplitude or the intensity in each depth, blood flow information is generated. As a scanning method for the flow mode, sequential alternating scan is known; the set scan area is divided into multiple partial areas, scanning is sequentially conducted in scan directions (lines) in each partial area, and scanning is repeated multiple times in this scan order so that ultrasound echoes are acquired in each scan direction (for example, see Patent Literature 1). According to Patent Literature 1, during sequential alternating scan for a flow image, when a partial area as the scan target is changed, a dummy beam is transmitted to prevent the discontinuity of a receiving condition between scan directions that are apart from each other in space, which occurs when scanning is repeated.

### Citation List

### Patent Literature

Patent Literature 1: Japanese Laid-open Patent Publication No. 2006-198075

### Summary

### Technical Problem

Unfortunately, with regard to the sequential alternating scan disclosed in Patent Literature 1, no consideration is given to residual echoes that occur during scan. Therefore, when scanning is conducted in a partial area, noise due to residual echoes is included in two-dimensional information, and superimposed images are sometimes unclear images.

The present invention has been made in consideration of the foregoing, and it has an object to provide an ultrasound observation device, a method for operating the ultrasound observation device, and a program for operating the ultrasound observation device that makes it possible to prevent effects of noise due to residual echoes during sequential alternating scan.

### Solution to Problem

To solve the problem described above and to achieve the object, an ultrasound observation device according to the present invention is an ultrasound observation device configured to generate Doppler information based on multiple sets of scan data acquired during sequential alternating scan in which ultrasound waves are sequentially transmitted in multiple depth directions and in which the ultrasound waves are repeatedly transmitted in this sequential order. The ultrasound observation device includes: a calculation unit configured to perform comparison operation on first scan data acquired by transmitting the ultrasound waves in a depth direction for first scan during the sequential alternating scan and second scan data acquired by transmitting the ultrasound waves for a second time during the sequential alternating scan, the second scan data being acquired by transmitting the ultrasound waves in a depth direction identical to the depth direction for the first scan; and a sequential-alternating scan controller configured to control a repetition frequency for repeatedly transmitting the ultrasound waves during the sequential alternating scan based on a calculation result of the calculation unit.

In the ultrasound observation device according to the present invention, the calculation unit further includes: a subtracting unit configured to conduct subtraction on the first scan data and the second scan data; a determining unit configured to compare a subtraction result of the subtracting unit with a threshold to determine presence or absence of a residual echo in the second scan data; and a repetition-frequency setting unit configured to change a setting of the repetition frequency when the determining unit determines that there is the residual echo in the second scan data.

In the ultrasound observation device according to the present invention, the repetition-frequency setting unit is configured to calculate an added time for setting the repetition frequency based on the residual echo detected.

In the ultrasound observation device according to the present invention, as for a time interval for ultrasound transmission, the repetition-frequency setting unit is configured to set the repetition frequency such that the time interval for ultrasound transmission is a time equal to or more than a time obtained by adding the added time to a set time interval for ultrasound transmission.

In the ultrasound observation device according to the present invention, the determining unit is configured to detect a peak of a residual echo by comparing the subtraction result with a threshold.

In the ultrasound observation device according to the present invention, a scan area for generating the Doppler information is divided into partial areas, the sequential-alternating scan controller is configured to perform control to conduct the sequential alternating scan in each of the partial areas, and the calculation unit is configured to perform the comparison operation in one or more specified partial areas.

In the ultrasound observation device according to the present invention, the repetition-frequency setting unit is configured to set the repetition frequency by changing a number of scan lines for conducting the sequential alternating scan.

In the ultrasound observation device according to the present invention, the sequential-alternating scan controller is configured to perform control to set the repetition frequency for the sequential alternating scan on a per frame basis.

In the ultrasound observation device according to the present invention, before the sequential alternating scan is conducted, the sequential-alternating scan controller is configured to perform an advance process in which the ultrasound waves are transmitted in a depth direction for first scan during the sequential alternating scan, the ultrasound waves are transmitted in a depth direction for last scan during the sequential alternating scan, and then the ultrasound waves are transmitted in a depth direction identical to the depth direction for the first scan, the calculation unit is configured to perform comparison operation on the first scan data and the second scan data acquired during the advance process, and the sequential-alternating scan controller is configured to control the repetition frequency for the sequential alternating scan in accordance with a calculation result of the calculation unit using scan data based on the advance process.

A method for operating an ultrasound observation device according to the present invention is a method for operating an ultrasound observation device configured to generate Doppler information based on multiple sets of scan data acquired during sequential alternating scan in which ultrasound waves are sequentially transmitted in multiple depth directions and in which the ultrasound waves are repeatedly transmitted in this sequential order. The method includes: by a calculation unit, a calculation step of performing comparison operation on first scan data acquired by transmitting the ultrasound waves in a depth direction for first scan during the sequential alternating scan and second scan data acquired by transmitting the ultrasound waves for a second time during the sequential alternating scan, the second scan data being acquired by transmitting the ultrasound waves in a depth direction identical to the depth direction for the first scan; and by a sequential-alternating scan controller, a sequential-alternating scan control step of controlling a repetition frequency for repeatedly transmitting the ultrasound waves during the sequential alternating scan based on a calculation result of the calculation unit.

A program for operating an ultrasound observation device according to the present invention is a program for operating an ultrasound observation device configured to generate Doppler information based on multiple sets of scan data acquired during sequential alternating scan in which ultrasound waves are sequentially transmitted in multiple depth directions and in which the ultrasound waves are repeatedly transmitted in this sequential order. The program causes the ultrasound observation device to execute: a calculation step of performing comparison operation on first scan data acquired by transmitting the ultrasound waves in a depth direction for first scan during the sequential alternating scan and second scan data acquired by transmitting the ultrasound waves for a second time during the sequential alternating scan, the second scan data being acquired by transmitting the ultrasound waves in a depth direction identical to the depth direction for the first scan; and a sequential-alternating scan control step of controlling a repetition frequency for repeatedly transmitting the ultrasound waves during the sequential alternating scan based on a calculation result at the calculation step.

### Advantageous Effects of Invention

According to the present invention, there is an advantage such that it is possible to prevent effects of noise due to residual echoes during sequential alternating scan.

### Brief Description of Drawings

FIG. 1 is a block diagram that illustrates a configuration of an ultrasound diagnosis system including an ultrasound observation device according to an embodiment of the present invention.
FIG. 2 is a diagram that illustrates an example of a region of interest that is set by the ultrasound observation device according to an embodiment of the present invention.
FIG. 3 is a diagram that illustrates an example of the ultrasound transmission timing when the ultrasound observation device according to an embodiment of the present invention acquires RF data for flow.
FIG. 4 is a diagram that illustrates a reception echo during acquisition of flow RF data conducted by the ultrasound observation device according to an embodiment of the present invention.
FIG. 5 is a diagram that illustrates an ultrasound transmission process under the control of the ultrasound observation device according to an embodiment of the present invention.
FIG. 6 is a flowchart that illustrates a process to set the PRF, conducted by the ultrasound observation device according to an embodiment of the present invention.
FIG. 7 is a diagram that illustrates a change in the setting of the PRF, conducted by the ultrasound observation device according to an embodiment of the present invention.
FIG. 8 is a diagram that illustrates a change in the setting of the PRF, conducted by the ultrasound observation device according to an embodiment of the present invention.
FIG. 9 is a diagram that illustrates a process during a flow mode, conducted by the ultrasound observation device according to a modification 1 of the embodiment of the present invention.
FIG. 10 is a diagram that illustrates a process during a flow mode, conducted by the ultrasound observation device according to the modification 1 of the embodiment of the present invention.
FIG. 11 is a diagram that illustrates a process during a flow mode, conducted by the ultrasound observation device according to a modification 2 of the embodiment of the present invention.

### Description of Embodiments

With reference to attached drawings, an explanation is given below of an aspect (hereafter, referred to as "embodiment") for implementing the present invention. Furthermore, the embodiment described below is not a limitation on the present invention. Moreover, in description of the drawings, the same components are attached with the same reference numeral.

### (Embodiment)

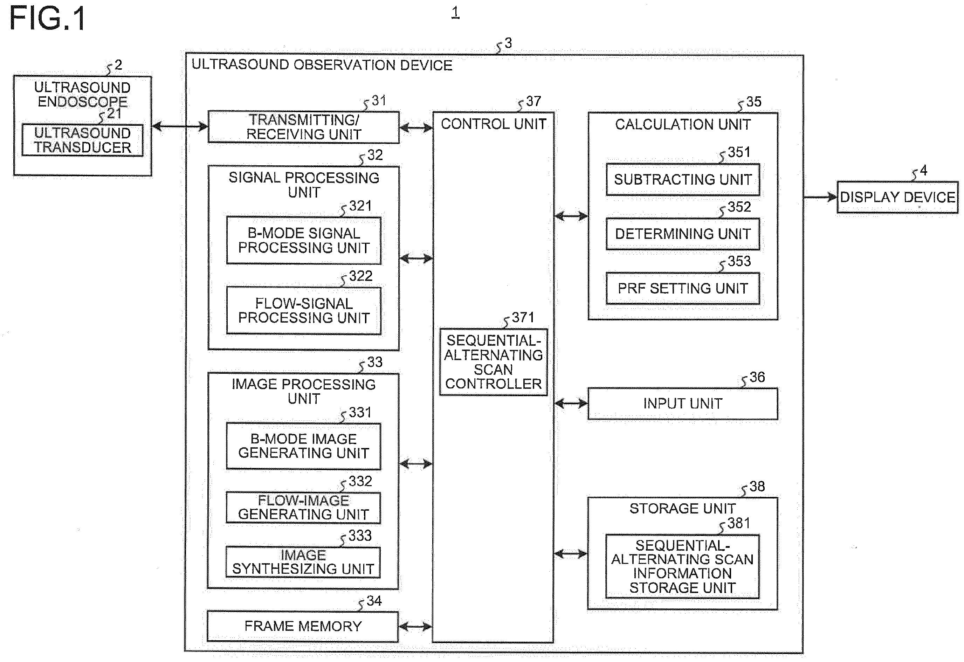

FIG. 1 is a block diagram that illustrates a configuration of an ultrasound diagnosis system including an ultrasound observation device according to an embodiment of the present invention. An ultrasound diagnosis system 1 illustrated in the drawing includes an ultrasound endoscope 2 that transmits ultrasound waves to the subject, which is an observation target, and receives ultrasound waves reflected by the subject; an ultrasound observation device 3 that generates ultrasound images based on ultrasound signals acquired by the ultrasound endoscope 2; and a display device 4 that displays ultrasound images generated by the ultrasound observation device 3.

At the distal end of the ultrasound endoscope 2, an ultrasound transducer 21 is provided which converts electric pulse signals received from the ultrasound observation device 3 into ultrasound pulses (sound pulses) and emits them to the subject and also converts ultrasound echoes reflected by the subject into electric echo signals (ultrasound signals) represented by changes in a voltage and outputs them. The ultrasound transducer 21 is implemented by using a radial-type transducer. The ultrasound endoscope 2 may cause the ultrasound transducer 21 to conduct scanning mechanically or may cause it to conduct scanning electronically with elements arranged in array as the ultrasound transducer 21 by electronically switching elements for transmitting/receiving or by applying a delay for each element in transmitting/receiving.

The ultrasound endoscope 2 typically includes an optical imaging system and an imaging element, and it is inserted into a digestive tract (esophagus, stomach, duodenum, large intestine) or respiratory apparatus (trachea, bronchi) of the subject so as to capture the digestive tract, respiratory apparatus, or their periphery organs (pancreas, gallbladder, bile duct, biliary tract, lymph node, mediastinal organ, blood vessel, or the like). Furthermore, the ultrasound endoscope 2 includes a light guide that guides illumination light emitted to the subject during capturing. The distal end of the light guide reaches the distal end of the insertion unit of the ultrasound endoscope 2 for the subject while the proximal end thereof is connected to a light source device that generates the illumination light.

The ultrasound observation device 3 includes a transmitting/receiving unit 31, a signal processing unit 32, an image processing unit 33, a frame memory 34, a calculation unit 35, an input unit 36, a control unit 37, and a storage unit 38.

The transmitting/receiving unit 31 is electrically connected to the ultrasound endoscope 2 so that it transmits transmission signals (pulse signals) that are high-voltage pulses based on a predetermined waveform and transmission timing to the ultrasound transducer 21 and receives echo signals, which are electric reception signals, from the ultrasound transducer 21 to generate and output digital high-frequency (RF: Radio Frequency) signal data (hereafter, referred to as RF data).

The frequency band of pulse signals transmitted by the transmitting/receiving unit 31 is preferably a wide band so as to almost cover the linear-response frequency band for electroacoustic conversion from pulse signals to ultrasound pulses by the ultrasound transducer 21.

The transmitting/receiving unit 31 has a function to transmit various control signals output from the control unit 37 to the ultrasound endoscope 2, and it also has a function to receive various types of information including ID for identification from the ultrasound endoscope 2 and transmit it to the control unit 37.

Furthermore, the transmitting/receiving unit 31 transmits transmission signals (pulse signals), which are high-voltage pulses, to the ultrasound transducer 21 in accordance with predetermined transmission timing under the control of the control unit 37 depending on an image generated, either a B-mode image that corresponds to an echo signal or a flow image that is two-dimensional Doppler information in which the presence or absence of blood flow or the direction of blood flow is color-coded. Specifically, when a flow image is generated, for example, the transmitting/receiving unit 31 transmits pulses at ultrasound transmission timing for flow. The transmitting/receiving unit 31 transmits ultrasound waves multiple times in an identical direction and receives reflected echo signals, thereby acquiring echo signals for flow. After receiving echo signals for flow, the transmitting/receiving unit 31 generates RF data for flow and outputs it to the signal processing unit 32.

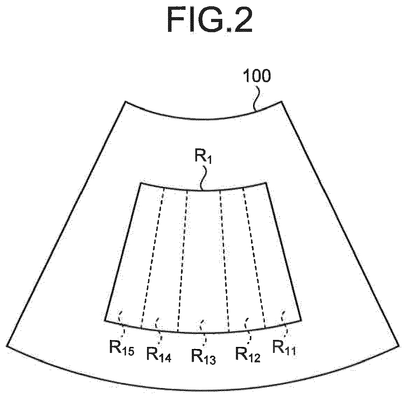

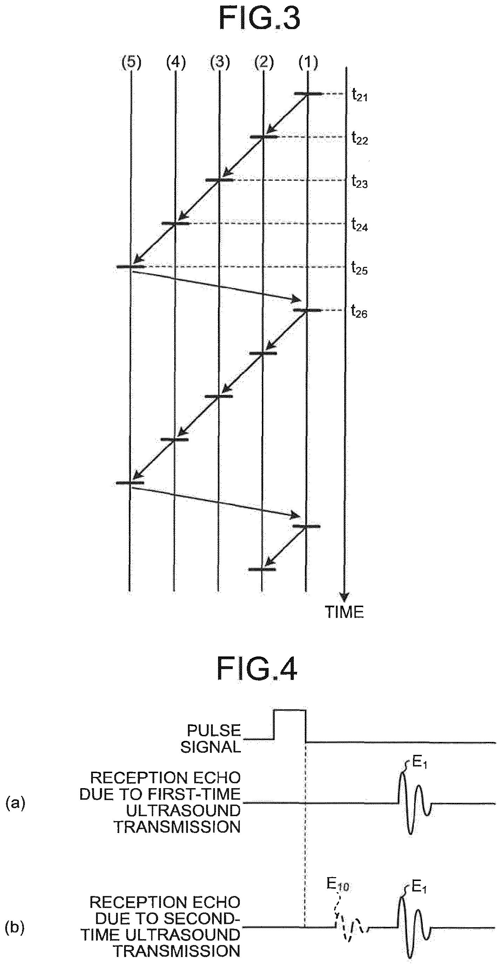

FIG. 2 is a diagram that illustrates an example of a region of interest that is set by the ultrasound observation device according to an embodiment of the present invention. FIG. 3 is a diagram that illustrates an example of the ultrasound transmission timing when the ultrasound observation device according to an embodiment of the present invention acquires RF data for flow. When a flow image is acquired according to the present embodiment, echo signals are acquired during sequential alternating scan. During the sequential alternating scan, scanning is conducted multiple times on scan lines that are set in each of partial areas (partial areas R₁₁ to R₁₅, hereafter also referred to as blocks) that are areas divided from a region of interest R₁ that is set in a scan area 100 illustrated in FIG. 2. For example, when five scan lines (e.g., a first scan line, a second scan line, ..., a fifth scan line) are set in a certain partial area, scan is sequentially conducted, starting from the first scan line until the fifth scan line, and then the first scan line is returned again so that echo signals are acquired for each scan line. Specifically, as illustrated in FIG. 3, first-time scan is conducted on the first scan line (1) at time t₂₁, first-time scan is then conducted on the second scan line (2) at time t₂₂, first-time scan is conducted on the third scan line (3) at time t₂₃, first-time scan is conducted on the fourth scan line (4) at time t₂₄, and first-time scan is conducted on the fifth scan line (5), which is the last scan line, at time t₂₅. Then, second-time scan is conducted on the first scan line at time t₂₆, and sequentially second-time scan is performed. During the sequential alternating scan, scanning is conducted for the set number of times to acquire echo signals for each scan line. Furthermore, as for scan timing during the sequential alternating scan, a scan interval is constant at least for the identical scan line.

For the region of interest R₁, for example, a trapezoidal-shaped or fan-like area is set. Furthermore, the region of interest may be a fan-like area that is surrounded by line segments that are parallel in a depth direction (sound ray direction) of the ultrasound transducer 21 and a curved line that connects ends of the line segments and that connects positions with the same depth from the surface of the ultrasound transducer 21. The "curved line" mentioned here is equivalent to a scan direction of the ultrasound transducer 21.

The signal processing unit 32 generates digital B-mode reception data and flow reception data on the basis of flow RF data received from the transmitting/receiving unit 31. The signal processing unit 32 includes a B-mode signal processing unit 321 that generates B-mode reception data; and a flow-signal processing unit 322 that generates flow reception data.

The B-mode signal processing unit 321 performs known processing such as bandpass filtering, envelope detection, or logarithmic conversion on RF data to generate digital B-mode reception data. For logarithmic conversion, the common logarithm of the amount of division of RF data by the reference voltage is represented by a decibel value. B-mode reception data is made up of multiple sets of line data in which the amplitude or the intensity of a reception signal indicating the intensity of reflection of an ultrasound pulse is arranged along a transmitting/receiving direction (depth direction) of the ultrasound pulse. The signal processing unit 32 outputs generated B-mode reception data corresponding to one frame to the image processing unit 33.

In the same manner, the flow-signal processing unit 322 performs the above-described process to generate flow reception data that is made up of multiple sets of line data on the basis of flow RF data. The flow-signal processing unit 322 uses RF data in the identical direction to calculate changes in the amplitude or the intensity of a reception signal indicating the intensity of reflection of an ultrasound pulse for each predetermined depth and generates a sound ray (line data) having the calculated amount of change. Flow reception data is made up of multiple sets of line data in which the amount of change in the amplitude or the intensity of a reception signal indicating the intensity of reflection of an ultrasound pulse is arranged along a transmitting/receiving direction (depth direction) of the ultrasound pulse. Furthermore, after detecting the received flow RF data, the flow-signal processing unit 322 outputs the detected RF data to the frame memory 34. The signal processing unit 32 is implemented by using a CPU (Central Processing Unit), various arithmetic circuits, or the like.

The image processing unit 33 generates B-mode image data and flow image data on the basis of B-mode reception data and flow reception data received from the signal processing unit 32. The image processing unit 33 includes a B-mode image generating unit 331 that generates B-mode image data on the basis of B-mode reception data; a flow-image generating unit 332 that generates flow image data on the basis of flow reception data; and an image synthesizing unit 333 that superimposes flow image data with B-mode image data to synthesize images.

The B-mode image generating unit 331 performs signal processing using a known technology such as scan converter processing, gain processing, or contrast processing on B-mode reception data output from the signal processing unit 32 and decimates data that corresponds to a data step width defined based on the display range of an image on the display device 4, or the like, thereby generating B-mode image data. During scan converter processing, the scan direction of B-mode reception data is converted from the scan direction of ultrasound waves into the display direction of the display device 4. B-mode images are gray-scaled images in which the values of R (red), G (green), and B (blue), which are variables when the RGB color system is used as a color space, are identical.

Furthermore, the flow-image generating unit 332 generates flow image data on the basis of flow reception data that is received from the signal processing unit 32. Specifically, the flow-image generating unit 332 applies color information to each depth position in accordance with a relative amount of change in the set region of interest and interpolates color information at a missing position, thereby generating flow image data. Color information is Doppler information representing the presence or absence of blood flow or the direction of blood flow at each position, and it is information represented in color that is relatively defined in accordance with a rate in the amount of change in the area for which Doppler information is generated, e.g., a region of interest.

The B-mode image generating unit 331 and the flow-image generating unit 332 perform coordinates conversion on B-mode reception data and flow reception data from the signal processing unit 32 to rearrange a scan area so as to be properly represented in space and then performs an interpolation process on B-mode reception data and flow reception data to fill gaps in each piece of reception data, thereby generating B-mode image data and flow image data.

The image synthesizing unit 333 superimposes flow image data with the generated B-mode image data on the basis of the coordinate information, thereby generating image data for display.

The frame memory 34 is implemented by using for example a ring buffer, and it stores flow RF data on one line, detected by the signal processing unit 32, in chronological order. The frame memory 34 may store flow RF data on multiple lines in chronological order. In this case, when the frame memory 34 runs out of space (stores flow reception data on a predetermined number of lines), it overwrites the earliest flow RF data with the latest flow RF data so as to store the latest flow RF data on a predetermined number of lines in chronological order. Furthermore, the frame memory 34 may store B-mode reception data, B-mode image data, or flow image data.

The calculation unit 35 detects the presence or absence of residual echoes that occur during scan on each partial area and sets a repetition frequency (Pulse Repetition Frequency: PRF) for transmitting ultrasound waves in accordance with the presence or absence of residual echoes. According to the present embodiment, the setting of the PFR is equivalent to the setting of the number of scan lines. The calculation unit 35 includes a subtracting unit 351, a determining unit 352, and a PRF setting unit 353.

The subtracting unit 351 refers to the frame memory 34 with respect to a partial area and conducts subtraction on RF data on a scan line obtained during the first scan in the partial area and RF data on the same scan line during the second or subsequent scan to calculate subtraction data. For example, the subtracting unit 351 calculates the subtraction data between RF data on the first scan line during the first scan in the partial area R₁₁ and RF data on the first scan data during the second scan, i.e., the subtraction data including the difference after subtracting the first-time RF data on the first scan line from the second-time RF data on the first scan line at a predetermined time interval (depth interval). It is preferable that the subtracting unit 351 calculates subtraction data by using RF data after noise cancelling with a Doppler filter.

The determining unit 352 compares the difference calculated by the subtracting unit 351 with a threshold stored in the storage unit 38 and determines whether the second and subsequent scan data includes residual echoes due to ultrasound waves transmitted during scan on the scan line before the corresponding scan line. Specifically, an echo signal due to ultrasound waves transmitted during acquisition of the first-time RF data on the fifth scan line is sometimes received while the second-time RF data on the first scan line is acquired, and the echo signal is a residual echo. The residual echo is not supposed to be included in the second-time RF data on the first scan line, and it appears as noise during generation of a flow image.

FIG. 4 is a diagram that illustrates a reception echo during acquisition of flow RF data conducted by the ultrasound observation device according to an embodiment of the present invention. With regard to a reception echo of a scan line acquired due to an ultrasound wave transmitted for the first time in a partial area, e.g., the reception echo illustrated in FIG. 4(a), no residual echo is received and an echo E₁ that corresponds to a blood flow is received as scan has not been previously conducted, i.e., the ultrasound transducer 21 has not scanned a different scan line. Conversely, with regard to the reception echo of a scan line acquired due to the ultrasound wave transmitted for the second or subsequent time, e.g., the second-time reception echo illustrated in FIG. 4(b), a residual echo E₁₀ due to the previously transmitted ultrasound wave, e.g., the ultrasound wave transmitted during acquisition of the first-time RF data on the fifth scan line, is sometimes received in addition to the above-described echo E₁. In this case, subtraction data calculated by the subtracting unit 351 has a waveform with a peak that corresponds to the residual echo. The determining unit 352 compares the above-described subtraction data with the threshold to detect the peak, thereby determining the presence or absence of a residual echo. The threshold used here is a value determined based on the peak value that may be detected as a residual echo. Furthermore, a residual echo occurs between adjacent scan lines; however, as they are close in space, they are represented in a continuous manner on a flow image. Conversely, when they are apart in space like the first scan line and the fifth scan line, they are represented discontinuously on a flow image; therefore, according to the present embodiment, the determining unit 352 detects a residual echo that is detected when they are apart in space.

The PRF setting unit 353 sets the PRF in accordance with a determination result of the determining unit 352. Specifically, when the determining unit 352 determines that a residual echo is included, the PRF setting unit 353 changes the setting of the PRF. The PRF setting unit 353 outputs the set PRF to the control unit 37.

The input unit 36 is implemented by using a user interface such as keyboard, mouse, trackball, or touch panel, and it receives input of various types of information. The input unit 36 outputs received information to the control unit 37.

The control unit 37 performs overall control of the ultrasound diagnosis system 1. The control unit 37 is implemented by using a CPU, various arithmetic circuits, or the like, which have calculation and control functions. The control unit 37 reads information, stored and saved in the storage unit 38, from the storage unit 38 and performs various types of arithmetic processing related to the method for operating the ultrasound observation device 3, thereby integrally controlling the ultrasound observation device 3. Furthermore, the control unit 37 may be configured by using a CPU that is shared by the signal processing unit 32.

The control unit 37 includes a sequential-alternating scan controller 371 that generates transmission signals (pulse signals) including information for setting the number of scan lines in a partial area and setting the transmission timing of ultrasound waves, and the like, on the basis of the PRF set by the PRF setting unit 353 during the sequential alternating scan conducted for acquiring flow images so as to control the sequential alternating scan.

The storage unit 38 stores data, and the like, including various programs for operating the ultrasound diagnosis system 1 and various parameters needed for operation of the ultrasound diagnosis system 1. The storage unit 38 includes a sequential-alternating scan information storage unit 381 that stores sequential-alternating scan information that is information related to the settings of sequential alternating scan. As for the sequential-alternating scan information, the above-described determining unit 352 stores a threshold (peak information) for determining the presence or absence of residual echoes and information related to the PRF that is set by the PRF setting unit 353.

Furthermore, the storage unit 38 stores various programs including an operation program for implementing a method of operating the ultrasound diagnosis system 1. The operation program may be widely distributed by being recorded in a recording medium readable by a computer, such as hard disk, flash memory, CD-ROM, DVD-ROM, or flexible disk. Furthermore, the above-described various programs may be acquired by being downloaded via a communication network. The communication network mentioned here is implemented by using, for example, an existing public network, LAN (Local Area Network), or WAN (Wide Area Network), and it may be wired or wireless.

The storage unit 38 having the above-described configuration is implemented by using a ROM (Read Only Memory) that has various programs, or the like, previously installed, a RAM (Random Access Memory) that stores calculation parameters, data, and the like, for each process, or the like.

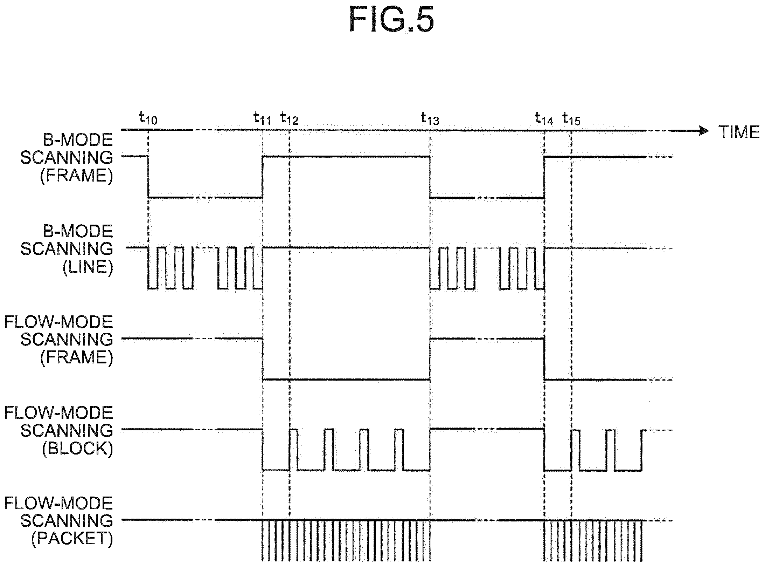

Next, an explanation is given of a process to generate B-mode image data and flow image data, conducted by the ultrasound observation device 3 according to the present embodiment. FIG. 5 is a diagram that illustrates an ultrasound transmission process under the control of the ultrasound observation device according to an embodiment of the present invention. Here, in explanation according to the present embodiment, for B-mode scanning, line scanning is conducted during the pulse falling or rising time.

For flow observation, flow-mode scanning is conducted to acquire flow echo signals in the interval between frames for B-mode scanning. In explanation for the flow-mode scanning illustrated in FIG. 5, flow-mode line scanning is conducted five times in each partial area. Here, signals in a group, obtained during this successive line scanning, are called a packet, and a packet is formed for each of the above-described partial areas.

Specifically, during the ultrasound transmission process according to the present embodiment, B-mode scanning corresponding to one frame is finished in the interval between, for example, time t₁₀ and time t₁₁, and flow-mode scanning is started at the time t₁₁. An ultrasound transmission process is performed for a single partial area in the interval between the time t₁₁ and the time t₁₂. For example, in the interval between the time t₁₁ and the time t₁₂, an ultrasound transmission process is performed for the area that corresponds to the partial area R₁₁ among the partial areas R₁₁ to R₁₅. In each partial area, line scanning is conducted on scan lines, e.g., from the first scan line, the second scan line, ..., and the fifth scan line, to acquire the reception echo that corresponds to one packet, and then line scanning (packet acquisition) for scan lines from the first scan line, the second scan line, ..., and the fifth scan line is repeated for a predetermined number of times. Then, an ultrasound transmission process is sequentially performed for other partial areas. After line scanning for the last scan line in the last partial area is finished at time t₁₃, the flow-mode reception echo corresponding to one frame may be acquired.

After the flow-mode scanning is finished, the above-described process is repeated so that B-mode scanning for the second frame is started from the time t₁₃ to time t₁₄, and after the B-mode scanning is finished, line scanning is conducted on the first partial area during flow-mode scanning for the second frame in the interval between the time t₁₄ and time t₁₅. In this way, by repeating B-mode scanning corresponding to one frame and flow-mode scanning corresponding to one frame, a reception echo is acquired to generate each piece of image information.

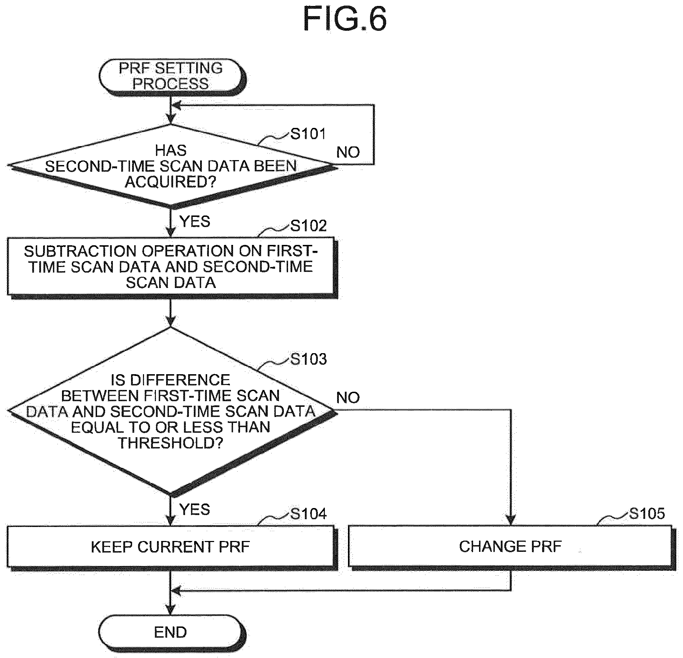

According to the present embodiment, when the calculation unit 35 performs a process to determine the presence or absence of a residual echo after the second-time RF data on the first scan line is acquired in a partial area, for example, and determines that there is a residual echo, it performs a PRF setting process (calculation step). FIG. 6 is a flowchart that illustrates a process to set the PRF, conducted by the ultrasound observation device according to an embodiment of the present invention. In the following explanation, each unit operates under the control of the control unit 37. First, the calculation unit 35 determines whether the second-time RF data (hereafter, also referred to as scan data) on a scan line in the partial area targeted for scanning has been acquired. When it is determined that the second-time scan data on a scan line has not been acquired (Step S101: No), the calculation unit 35 repeatedly checks scan data. Conversely, when it is determined that the second-time scan data on a scan line has been acquired (Step S101: Yes), the calculation unit 35 proceeds to Step S102.

At Step S102, the subtracting unit 351 performs a subtraction operation on RF data on the first scan line scanned for the first time (initially) in a partial area and RF data on the first scan line scanned for the second time, thereby calculating subtraction data.

After the difference is calculated, the determining unit 352 compares the difference calculated by the subtracting unit 351 with the threshold stored in the storage unit 38 to determine whether the second-time scan data includes a residual echo (Step S103). When the difference is equal to or less than the threshold and the determining unit 352 determines that the second-time scan data includes no residual echo (Step S103: Yes), it proceeds to Step S104. Conversely, when the difference is more than the threshold and the determining unit 352 determines that the second-time scan data includes a residual echo (Step S103: No), it proceeds to Step S105.

After determination by the determining unit 352, the PRF setting unit 353 sets the PRF for the subsequent sequential alternating scan in accordance with a determination result. Furthermore, after the PRF setting unit 353 sets the PRF, the sequential-alternating scan controller 371 performs sequential alternating scan to which the set PRF is applied (sequential-alternating scan control step).

At Step S104, as the second-time scan data does not include any residual echo, the PRF setting unit 353 keeps the current PRF. Thus, flow echo signals may be received without being affected by residual echoes while keeping the frame rate.

Conversely, at Step S105, as the second-time scan data includes a residual echo, the PRF setting unit 353 sets the PRF such that the residual echo is not included. The PRF setting unit 353 changes the PRF such that the third and subsequent RF data on the first scan line does not include any residual echoes while the reception interval of echo signals in each scan line is retained. Thus, although the frame rate is slightly reduced, echo signals may be acquired without being affected by residual echoes.

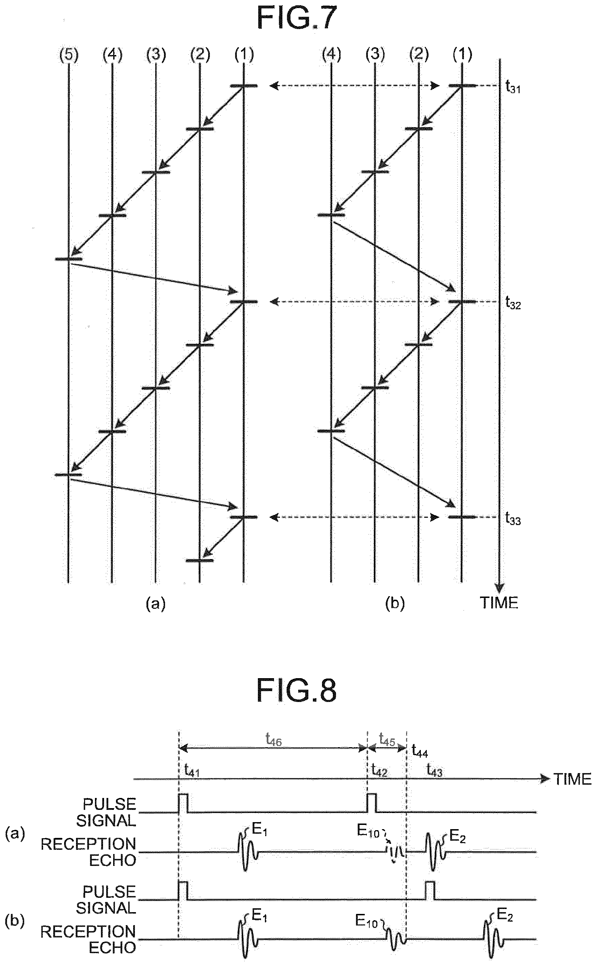

FIGS. 7 and 8 are diagrams that illustrate a change in the setting of the PRF, conducted by the ultrasound observation device according to an embodiment of the present invention. As illustrated in for example FIG. 7(a) and FIG. 7(b), the PRF setting unit 353 changes the setting of the PRF by decreasing the number of scan lines to increase the ultrasound transmission interval between adjacent scan lines while keeping the interval between time t₃₁ and time t₃₂ and the interval between time t₃₂ and time t₃₃, which is the reception interval between echo signals in the same scan line.

Before the PRF is changed (FIG. 8(a)), the residual echo E₁₀ occurring due to the ultrasound wave transmitted at time t₄₁, which is an ultrasound transmission time for the first-time line scanning on the last scan line, is present after time t₄₂ that is an ultrasound transmission start time of the scan line for the second time to acquire an echo E₂. Conversely, after the PRF is changed (FIG. 8(b)), the residual echo E₁₀ occurring due to the ultrasound wave transmitted at the time t₄₁, which is an ultrasound transmission time for the first-time line scanning on the last scan line, is present before time t₄₃ that is an ultrasound transmission time of the scan line for the second time. In this way, the process to change the PRF by the PRF setting unit 353 allows the first and subsequent RF data (reception echo) on the first scan line to be free from residual echoes.

Here, to change the PRF, as illustrated in FIG. 8, the PRF setting unit 353 counts the time until the residual echo E₁₀ is eliminated, i.e., the PRF setting unit 353 counts time t₄₅ from the time t₄₂ to time t₄₄, by using the time t₄₂, which is an ultrasound transmission start time for the second-time line scanning on the first scan line, as a base point, adds the time t₄₅ to time t₄₆ that is a count value from the time t₄₁, which is an ultrasound transmission start time for the line scanning on the last scan line, to the time t₄₂ to calculate the shortest time (t₄₅+t₄₆) per scan, and calculates the PRF, which is a repetition frequency, based on the shortest time. It is appropriate if the ultrasound transmission interval after the PRF is changed is equal to or more than the shortest time and, for example in FIG. 8(b), it is the interval between the time t₄₁ and the time t₄₃. In this way, the PRF setting unit 353 sets the PRF in accordance with the temporal position of a residual echo.

Furthermore, detection of residual echoes and changing of the PRF may be performed when sequential alternating scan in each partial area is conducted, that is, on a per frame basis, or when sequential alternating scan in one or more specified partial areas is conducted. If detection of residual echoes and changing of the PRF are performed when sequential alternating scan in one or more specified partial areas is conducted, the previously set PRF is retained and sequential alternating scan is conducted on partial areas for which detection of residual echoes or changing of the PRF is not conducted. An appropriate PRF may be set for each partial area by detecting residual echoes and changing the PRF when sequential alternating scan is conducted on each partial area (on a per frame basis) each time. Conversely, the number of calculations may be reduced by detecting residual echoes and changing the PRF when sequential alternating scan is conducted on one or more specified partial areas as compared with a case where it is conducted each time.

Then, by using reception signals of the respective scan lines obtained during flow-mode line scanning, the flow-signal processing unit 322 generates flow reception signal data including the amount of change in blood in the scan area. Here, blood flow information is generated from reception signals in accordance with the amount of change in the amplitude or the intensity for each depth. Furthermore, during flow-mode line scanning, after receiving the amplitude or the intensity in all depths for each line, blood flow information on the region may be acquired by using the set region of interest (ROI) as a scan area, or blood flow information may be acquired by using the entire image as a scan area.

The flow-image generating unit 332 generates flow image data on the basis of flow reception data received from the signal processing unit 32. The image synthesizing unit 333 superimposes the flow image data generated by the flow-image generating unit 332 with the B-mode image data generated by the B-mode image generating unit 331, thereby generating synthesis image data in which blood flow information is superimposed on the B-mode image data.

According to the above-described embodiment of the present invention, residual echoes are detected by comparing the first-time scan data on a scan line with the second or subsequent scan data on the scan line, that is, the scan data on the same scan line as the scan line for the first time, and when a residual echo is detected, the PRF, which is a repetition frequency, is changed; thus, when flow images are generated, it is possible to prevent effects of residual echoes between sets of scan data that are spatially apart. That is, according to the present embodiment, it is possible to prevent effects of noise on flow images due to residual echoes during sequential alternating scan.

### (Modification 1 of the embodiment)

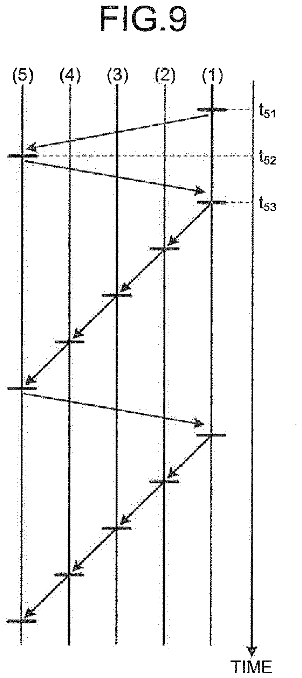

In explanation according to the above-described embodiment, residual echoes are detected by using the second-time scan data on scan lines that are repeatedly performed after scanning all the scan lines, the first scan line, the second scan line, ..., and the fifth scan line; according to a modification 1, residual echoes are detected by conducting scanning for detection before sequential alternating scan is performed. FIG. 9 is a diagram that illustrates a process during a flow mode, conducted by the ultrasound observation device according to the modification 1 of the embodiment of the present invention, and it is a diagram that illustrates sequential alternating scan when the determining unit 352 determines that no residual echo is included.

According to the modification 1, the sequential-alternating scan controller 371 first acquires scan data on the first scan line in a partial area at time t₅₁, acquires scan data on the last scan line at time t₅₂, and then acquires the second-time scan data on the first scan line at time t₅₃, and, as described above, the subtracting unit 351 calculates the subtraction data between RF data on the first scan line that is scanned for the first time and RF data on the first scan data that is scanned for the second time. Then, the determining unit 352 uses the subtraction data to detect a residual echo.

As illustrated in FIG. 9, when there are no residual echoes, the PRF is not changed, and therefore scan data on the first to the fifth scan lines is acquired during sequential alternating scan.

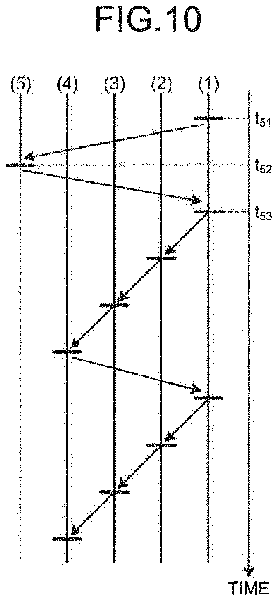

FIG. 10 is a diagram that illustrates a process during a flow mode, conducted by the ultrasound observation device according to the modification 1 of the embodiment of the present invention, and it is a diagram that illustrates sequential alternating scan when the determining unit 352 determines that a residual echo is included. As illustrated in FIG. 10, when there is a residual echo, the PRF is changed and, as described above, the scan lines are reduced. Therefore, during sequential alternating scan, the scan data on the first to the fourth scan lines are acquired. In this case, the time interval between the acquisition timing of the n time scan data and the acquisition timing of the n+1 time scan data is equal to the time interval between the acquisition timing of the n time scan data and the acquisition timing of the n+1 time scan data before the PRF is changed.

In this manner, before sequential alternating scan is conducted, the residual-echo detection process is performed and the PRF is previously changed; thus, particularly the time required for flow-mode scanning when a residual echo is detected may be shortened as compared with the time required for flow-mode scanning according to the above-described embodiment.

### (Modification 2 of the embodiment)

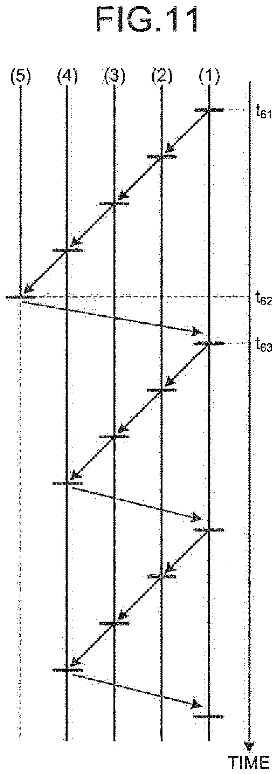

In explanation according to the above-described embodiment, after sequential alternating scan is started, a residual echo is detected, and the PRF is changed in accordance with a detection result; however, according to a modification 2, resetting is conducted when the PRF has been changed, and after the PRF is changed, sequential alternating scan is newly conducted by using the changed PRF. FIG. 11 is a diagram that illustrates a process during a flow mode, conducted by the ultrasound observation device according to the modification 2 of the embodiment of the present invention, and it is a diagram that illustrates sequential alternating scan when the determining unit 352 determines that a residual echo is included.

According to the modification 2, in the same manner as in the above-described embodiment, sequential alternating scan is conducted from time t₆₁, and after scan data on the first scan line in a partial area is acquired at the time t₆₁, sequential scan data is acquired, and after scan data on the last scan line is acquired at time t₆₂, the second-time scan data on the first scan line is acquired at time t₆₃. Then, the subtracting unit 351 calculates the subtraction data between the RF data on the first scan line scanned for the first time and the RF data on the first scan line scanned for the second time. Then, the determining unit 352 uses the subtraction data to detect a residual echo.

When there is a residual echo, the PRF is changed so that the scan lines are reduced as described above. In this case, according to the modification 2, the sequential alternating scan is reset at the time t₆₃, and sequential alternating scan is started at the time t₆₃ to newly acquire scan data on the first to the fourth scan lines.

In this way, when the residual-echo detection process is performed and the PRF is changed after the sequential alternating scan is started, resetting is performed so that sequential alternating scan is conducted with a new PRF; thus, it may be further ensured that flow images are obtained while preventing effects of residual echoes as compared with the flow-mode scanning according to the above-described embodiment.

Although the embodiment for implementing the present invention is explained above, the present invention should not be limited to only the above-described embodiment. For example, the ultrasound observation device may have a configuration such that circuits each having a function are connected via a bus or a configuration such that one function is installed in a circuit structure with another function.

Furthermore, in explanation according to the present embodiment, the ultrasound endoscope 2 including an optical system, such as a light guide, is used as an ultrasound probe; however, the ultrasound endoscope 2 is not a limitation, and an ultrasound probe that does not include any optical imaging system or imaging device may be used. Furthermore, an ultrasound miniature probe that is thin without any optical system may be used as the ultrasound probe. The ultrasound miniature probe is typically inserted into biliary tract, bile duct, pancreatic duct, trachea, bronchus, urethra, or ureter, and it is used for observation of a surrounding organ (pancreas, lung, prostate gland, urinary bladder, lymph node, or the like).

Furthermore, an external ultrasound probe that emits ultrasound waves from the body surface of the subject may be used as the ultrasound probe. The external ultrasound probe is typically used by being in direct contact with the body surface to observe abdominal organ (liver, gallbladder, urinary bladder), breast (specifically, mammary gland), or thyroid gland.

Furthermore, the ultrasound transducer may be a linear transducer, a radial transducer, or a convex transducer. When the ultrasound transducer is a linear transducer, its scan area is quadrilateral (rectangle, square), and when the ultrasound transducer is a radial transducer or a convex transducer, its scan area is a fan-like or ring-like shape. Furthermore, the ultrasound endoscope may cause the ultrasound transducer to conduct scanning mechanically or may cause it to conduct scanning electronically with multiple elements arranged in array as an ultrasound transducer by electronically switching the elements for transmitting/receiving or by delaying transmitting/receiving of each element.

In this way, the present invention may include various embodiments without departing from the technical idea disclosed in claims.

### Industrial Applicability

As described above, the ultrasound observation device, the method for operating the ultrasound observation device, and the program for operating the ultrasound observation device according to the present invention are advantageous in preventing effects of noise due to residual echoes during sequential alternating scan. Reference Signs List
1 ULTRASOUND DIAGNOSIS SYSTEM
2 ULTRASOUND ENDOSCOPE
3 ULTRASOUND OBSERVATION DEVICE
4 DISPLAY DEVICE
21 ULTRASOUND TRANSDUCER
31 TRANSMITTING/RECEIVING UNIT
32 SIGNAL PROCESSING UNIT
33 IMAGE PROCESSING UNIT
34 FRAME MEMORY
35 CALCULATION UNIT
36 INPUT UNIT
37 CONTROL UNIT
38 STORAGE UNIT
321 B-MODE SIGNAL PROCESSING UNIT
322 FLOW-SIGNAL PROCESSING UNIT
331 B-MODE IMAGE GENERATING UNIT
332 FLOW-IMAGE GENERATING UNIT
333 IMAGE SYNTHESIZING UNIT
351 SUBTRACTING UNIT
352 DETERMINING UNIT
353 PRF SETTING UNIT
371 SEQUENTIAL-ALTERNATING SCAN CONTROLLER
381 SEQUENTIAL-ALTERNATING SCAN INFORMATION STORAGE UNIT

## Claims

1. An ultrasound observation device configured to generate Doppler information based on multiple sets of scan data acquired during sequential alternating scan in which ultrasound waves are sequentially transmitted in multiple depth directions and in which the ultrasound waves are repeatedly transmitted in this sequential order, the ultrasound observation device comprising:
a calculation unit configured to perform comparison operation on first scan data acquired by transmitting the ultrasound waves in a depth direction for first scan during the sequential alternating scan and second scan data acquired by transmitting the ultrasound waves for a second time during the sequential alternating scan, the second scan data being acquired by transmitting the ultrasound waves in a depth direction identical to the depth direction for the first scan; and
a sequential-alternating scan controller configured to control a repetition frequency for repeatedly transmitting the ultrasound waves during the sequential alternating scan based on a calculation result of the calculation unit.

2. The ultrasound observation device according to claim 1, wherein the calculation unit further includes:
a subtracting unit configured to conduct subtraction on the first scan data and the second scan data;
a determining unit configured to compare a subtraction result of the subtracting unit with a threshold to determine presence or absence of a residual echo in the second scan data; and
a repetition-frequency setting unit configured to change a setting of the repetition frequency when the determining unit determines that there is the residual echo in the second scan data.

3. The ultrasound observation device according to claim 2, wherein the repetition-frequency setting unit is configured to calculate an added time for setting the repetition frequency based on the residual echo detected.

4. The ultrasound observation device according to claim 3, wherein, as for a time interval for ultrasound transmission, the repetition-frequency setting unit is configured to set the repetition frequency such that the time interval for ultrasound transmission is a time equal to or more than a time obtained by adding the added time to a set time interval for ultrasound transmission.

5. The ultrasound observation device according to claim 2, wherein the determining unit is configured to detect a peak of a residual echo by comparing the subtraction result with a threshold.

6. The ultrasound observation device according to claim 1, wherein
a scan area for generating the Doppler information is divided into partial areas,
the sequential-alternating scan controller is configured to perform control to conduct the sequential alternating scan in each of the partial areas, and
the calculation unit is configured to perform the comparison operation in one or more specified partial areas.

7. The ultrasound observation device according to claim 2, wherein the repetition-frequency setting unit is configured to set the repetition frequency by changing a number of scan lines for conducting the sequential alternating scan.

8. The ultrasound observation device according to claim 1, wherein the sequential-alternating scan controller is configured to perform control to set the repetition frequency for the sequential alternating scan on a per frame basis.

9. The ultrasound observation device according to claim 1, wherein
before the sequential alternating scan is conducted, the sequential-alternating scan controller is configured to perform an advance process in which the ultrasound waves are transmitted in a depth direction for first scan during the sequential alternating scan, the ultrasound waves are transmitted in a depth direction for last scan during the sequential alternating scan, and then the ultrasound waves are transmitted in a depth direction identical to the depth direction for the first scan,
the calculation unit is configured to perform comparison operation on the first scan data and the second scan data acquired during the advance process, and
the sequential-alternating scan controller is configured to control the repetition frequency for the sequential alternating scan in accordance with a calculation result of the calculation unit using scan data based on the advance process.

10. A method for operating an ultrasound observation device configured to generate Doppler information based on multiple sets of scan data acquired during sequential alternating scan in which ultrasound waves are sequentially transmitted in multiple depth directions and in which the ultrasound waves are repeatedly transmitted in this sequential order, the method comprising:
by a calculation unit, a calculation step of performing comparison operation on first scan data acquired by transmitting the ultrasound waves in a depth direction for first scan during the sequential alternating scan and second scan data acquired by transmitting the ultrasound waves for a second time during the sequential alternating scan, the second scan data being acquired by transmitting the ultrasound waves in a depth direction identical to the depth direction for the first scan; and
by a sequential-alternating scan controller, a sequential-alternating scan control step of controlling a repetition frequency for repeatedly transmitting the ultrasound waves during the sequential alternating scan based on a calculation result of the calculation unit.

11. A program for operating an ultrasound observation device configured to generate Doppler information based on multiple sets of scan data acquired during sequential alternating scan in which ultrasound waves are sequentially transmitted in multiple depth directions and in which the ultrasound waves are repeatedly transmitted in this sequential order, the program causing the ultrasound observation device to execute:
a calculation step of performing comparison operation on first scan data acquired by transmitting the ultrasound waves in a depth direction for first scan during the sequential alternating scan and second scan data acquired by transmitting the ultrasound waves for a second time during the sequential alternating scan, the second scan data being acquired by transmitting the ultrasound waves in a depth direction identical to the depth direction for the first scan; and
a sequential-alternating scan control step of controlling a repetition frequency for repeatedly transmitting the ultrasound waves during the sequential alternating scan based on a calculation result at the calculation step.
